(19) Europäisches Patentamt
European Patent Office
Office européen des brevets

(11) Publication number: **0 165 800**
**A1**

# (12) EUROPEAN PATENT APPLICATION

(21) Application number: **85304328.9**

(22) Date of filing: **17.06.85**

(51) Int. Cl.[4]: **C 08 B 37/00**
B 01 J 20/24, G 01 N 30/48
C 12 N 11/10, C 12 Q 1/54
B 01 D 15/08

(30) Priority: **20.06.84 GB 8415666**

(43) Date of publication of application:
**27.12.85 Bulletin 85/52**

(84) Designated Contracting States:
**DE FR GB IT SE**

(71) Applicant: **NATIONAL RESEARCH DEVELOPMENT CORPORATION**
**101 Newington Causeway**
**London SE1 6BU(GB)**

(72) Inventor: **Berezenko, Stephen**
**16 Dalziel Place**
**Edinburgh EH7 5TP Scotland(GB)**

(72) Inventor: **Sturgeon, Robert James**
**10 Eskview Grove**
**Dalkeith Midlothian EH22 1JW Scotland(GB)**

(74) Representative: **Percy, Richard Keith**
**Patent Department National Research Development Corporation 101 Newington Causeway**
**London SE1 6BU(GB)**

## (54) Support material for immobilisation of ligands.

(57) It has been a problem to find alternatives to agarose as a support material for affinity chromatography. It has now been found that a polysaccharide having pendant or terminal D-galactose residues can, on the one hand, be immobilised and on the other hand activated enzymically by D-galactose oxidase to form aldehyde groups, which are then reacted with a primary or secondary amino group of a ligand, for example an immunoglobulin. Immobilisation is by cross-linking or by a preceding, partial oxidation of the D-galactose residues to link the polysaccharide to a matrix through an amino group, whereby the D-galactose residues are used to perform two different functions.

The invention includes the support material *per se*, the polysaccharide, preferably a D-galacto-D-mannan, having pendant or terminal groups of formula

$$-G-CH_2-\underset{\displaystyle R}{\underset{|}{N}}-LIG$$

(G = a D-galactose residue, LIG = a ligand residue, R = H or R and LIG together represent a residue of the same ligand molecule), its preparation and its use in the affinity chromatography of a binding partner for the ligand.

EP 0 165 800 A1

Croydon Printing Company Ltd.

126861

# SUPPORT MATERIAL FOR IMMOBILISATION OF LIGANDS

## Background of the invention

### 1. Field of the invention

This invention relates to a support material for immobilisation of ligands, especially but not exclusively for affinity chromatography.

### 2. Description of prior art - Part A

In affinity chromatography the support material comprises an insoluble matrix having attached to it, normally covalently, a ligand which has an affinity for a binding partner which it is desired to purify. For example, protein A can be purified on a column having IgG attached to an insoluble matrix. Many different matrices have been proposed but for the purification of biological molecules, the choice is relatively restricted. Agarose is frequently used because it is hydrophilic, macroporous and stable. It is, however, expensive and requires chemical activation, e.g. by CNBr, for the binding of proteins thereto. Support materials having a silica matrix have recently been reviewed by S. Ohlson and M. Glad, in "Affinity Chromatography and Biological Recognition", ed. Chaiken, Wilchek and Parikh, Academic Press New York (1983), pages 241-250. These materials have various drawbacks described by the reviewers.

Additional description of prior art (Part B) follows the "Summary of the invention" without which the context of the additional prior art would not be clear.

## Summary of the invention

The present invention is based on the idea of using a completely different polysaccharide from agarose, several forms of which are commercially available in large quantities, and on the realisation that it can be both immobilised and activated to react with the amine-containing ligands with great ease. In the present invention the polysaccharide has many pendant or terminal (non-reducing) D-galactose groups. When they are activated by the enzyme D-galactose oxidase (EC 1.1.3.9), an aldehyde group is

formed. The aldehyde group can be reacted with an amine-containing ligand. To immobilise the polysaccharide it can be cross-linked or, in a particularly ingenious embodiment of the invention, some of the pendant or terminal D-galactose groups can be subjected to a preliminary oxidation by D-galactose oxidase and thereafter reaction with a matrix containing an amino group. The amount of enzyme used is deliberately limited, thereby leaving free D-galactose groups for further oxidation to activate them for reaction with the ligand.

In one aspect the invention provides a support material comprising an immobilised polysaccharide having pendant or terminal groups of general formula

$$-G-CH_2-\underset{\displaystyle R}{\underset{|}{N}}-LIG$$

wherein:

G represents a D-galactose residue

LIG represents a ligand residue, and

R represents a hydrogen atom,

or LIG and R together represent a residue of the same ligand molecule.

The preferred polysaccharide is a D-galacto-D-mannan, since it is conveniently available from guar gum as "guaran" and from other natural sources, as described hereinafter.

In another aspect, the invention provides a process of preparing a support material as defined above, the process comprising

(a) immobilising a polysaccharide having pendant or terminal non-reducing D-galactose residues thereon

(b) activating the immobilised polysaccharide by oxidation of $-CH_2OH$ groups present in the D-galactose residues by D-galactose oxidase, and

(c) reacting the activated immobilised polysaccharide with a ligand containing a primary or secondary amino group to form a Schiff's base (aldo-imine or -imminium ion), and with a reducing agent to reduce the Schiff's base.

Thirdly, the invention provides for the use of the above-defined support material in affinity chromatography. This can be expressed as a process of affinity chromatography, which comprises chromatographing a binding partner for the ligand on the support material.

Additional description of prior art (Part B)

What is novel and inventive in this invention comprises the idea of making a radical departure from agarose technology, and of how to make such a departure by immobilising a soluble polysaccharide of a different kind and of attaching ligand groups to it. The chemical reactions involved are either known or analogous to known processes. Thus, L.D. Hall and M. Yalpani in Carbohydrate Research 81, C10-C12 (1980) have described the chemical derivatisation of D-galacto-D-mannan. This polysaccharide was oxidised by D-galactose oxidase (EC 1.1.3.9) in order to introduce an aldehyde group into the pendant D-galactose residues. The aldehyde group was then reacted with the amino free radical 4-amino-2,2,6,6-tetramethyl-piperidin-1-oxyl and sodium cyanoborohydride, whereupon the amine became covalently bound to the aldehydic C-atom of the galactose residues, according to the reaction scheme

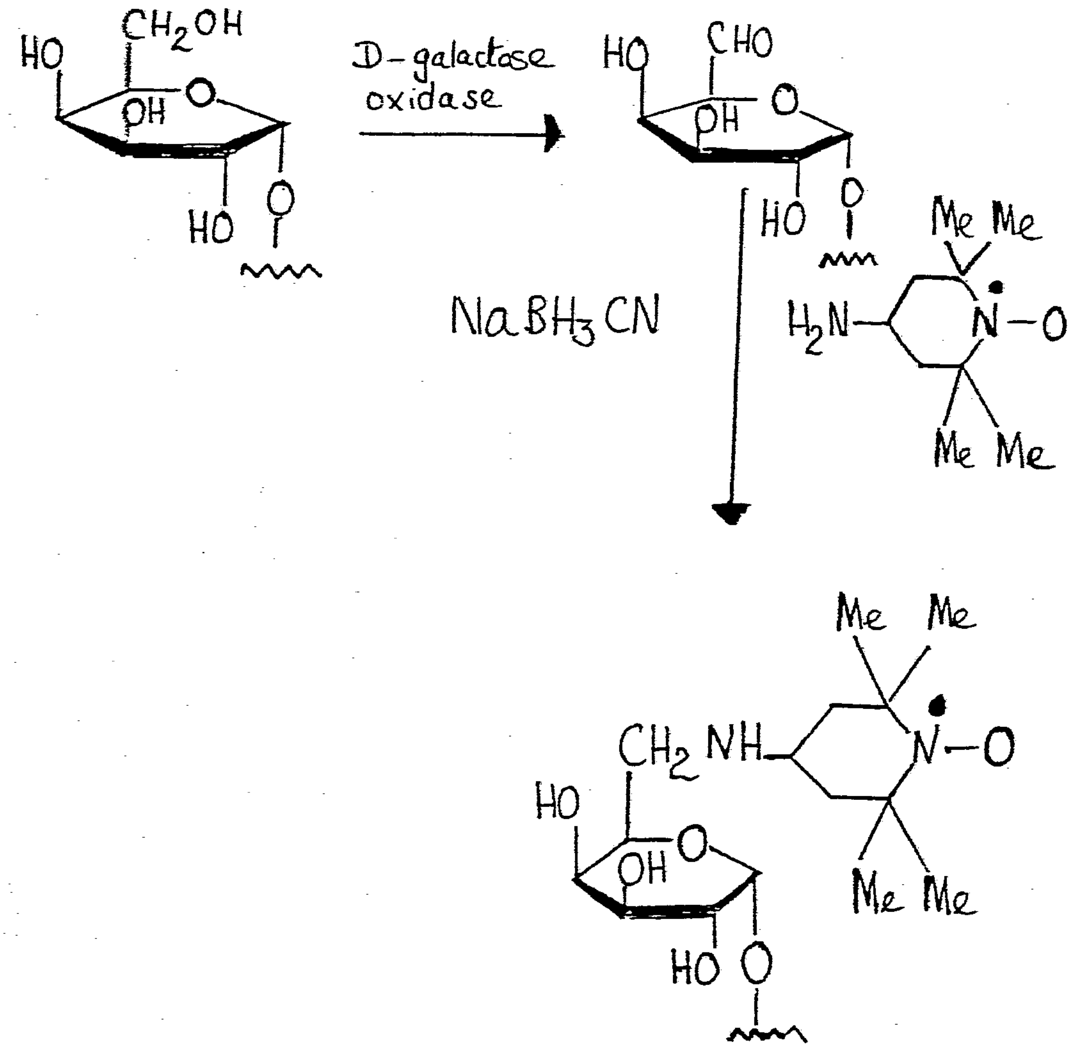


By this means the nitroxide radical groups are attached to the galactomannan in order to provide it with a label for the study of the molecule by electron spin resonance. While the authors of this paper drew attention to the potential use of this method for introducing a substituent of choice (by choosing another amine to react with the aldehyde), they did not suggest any practical purposes for so doing. The insolubilisation of D-galactomannan by cross-linking it is known from J. Lonngren, I.J. Goldstein and R. Bywater, Febs Letters, 68, pages 31-34 (1976), who used cross-linked guaran to purify certain lectins by affinity chromatography. (Lectins and alpha-D-galactopyranosyl groups exhibit a lock-and-key

binding interaction). The use of aminopropyl glass as an insoluble matrix for immobilising enzymes is mentioned by H.H. Weetall, Methods in Enzymology, 44, pages 134-148, but this paper does not describe any reaction of the glass with a polysaccharide.

It is known to coat porous glass with glycerylsilane, oxidise the vicinal -OH groups of the glyceryl residue with sodium periodate, react the oxidised supports with an enzyme such as trypsin, and reduce the resultant Schiff's base with sodium borohydride or cyanoborohydride, see G.P. Royer *et al*., Biochemical and Biophysical Research Communications, 64, pages 478-484 (1975). US Patent 4,430,348 describes attachment of glucoamylase by similar means, to a ceramic support.

An attempt has been made to modify agarose in order to use it for the affinity chromatography of the enzyme myoinositol oxygenase, an iron-containing enzyme which exists in various oligomeric states. Thus, F. Koller and E. Koller in Journal of Chromatography 283, pages 191-197 (1984) describe subjecting agarose to partial acid hydrolysis, oxidising the product with a mixture of peroxidase and galactose oxidase and using the product for chromatography of the enzyme. The chromatography only worked well by the addition of $Fe^{2+}$ ions. There is no suggestion of any covalent bonding of ligands to aldehyde groups.

While the above collection of literature known to the inventors indicates that several features of the invention are *per se* known, it is a collection made with the benefit of their having first had the idea of the present invention. This is to say, it is an *ex post facto* assembly of prior art. No one, to the inventors' knowledge, has previously suggested both immobilising galactomannan and modifying it for use in affinity chromatography.

## Description of the preferred embodiments

The invention is conveniently described by reference to D-galacto-D-mannan (hereinafter "galactomannan" for brevity) as the polysaccharide. However, it will be understood that the reactions thereof are applicable to other polysaccharides having

free galactose groups in which there is a 5-$CH_2OH$ function together with a terminal 1-carbon atom (as distinct from a linkage to another sugar residue via the 1-position O-atom).

Examples of other such polysaccharides are "amyloids" which are glucans having galactosylxylosyl side-chains on a (1→ 4)-beta-glucan backbone and are described in "Encyclopedia of Plant Physiology" New Series, Volume 13A "Plant Carbohydrates I", ed. by F.A. Loewus and W. Tanner, Springer-Verlag 1982, pages 430-431.

Galactomannans are most conveniently obtained from the seed of Leguminoseae species, especially from guar gum. They can also be obtained form microbial sources, but water-soluble plant galactomannans are preferred. A variety of galactomannans and sources thereof is described by I.C.M. Dea and A. Morrison in "Advances in Carbohydrate Chemistry and Biochemistry" 31, pages 241-312 (1975). Galactomannans have a mannose chain to which galactose groups are linked, via the 6-C-atom of the mannose and the 1-C-atom of the galactose, to above one half of the mannose residues.

The enzymic oxidation of galactomannan proceeds very easily at room temperature according to the partial equation $-CH_2OH + O_2 = -CHO + H_2O_2$. The hydrogen peroxide produced in this oxidation must be removed to prevent build-up of an inhibitory concentration thereof. Conveniently it is removed enzymically, e.g. by catalase or peroxidase, but it could also be removed electrochemically. It is possible to use an elevated temperature if desired, although care must be taken not to denature the enzyme. The amount of D-galactose oxidase used or time or temperature of incubation can be adjusted to provide any desired proportion of aldehyde groups.

The ligand amine employed can be any primary or secondary amine capable of providing a means of attaching an appropriate ligand residue. That is, the oxidised galactomannan is reacted with a ligand which is itself a primary or secondary amine of formula $LIG-NH_2$ or $LIG-\underset{R}{N}H$, respectively, where LIG and R are as

defined above. In this definition "LIG" means any molecular residue having therein a binding function which is specific to some other molecule so that it will interact with it in any manner useful for affinity chromatography of that other molecule. It will be appreciated that in order to introduce onto the activated galactomannan a molecule which has a desired binding function but lacks the required amine group, that molecule would have first to be reacted with a group providing the amine group. For example, a molecule having a group can be reacted with an alkylene diamine to provide an amine terminal group. The resultant amino-terminated molecule would then be regarded as of formula $\text{LIG-NH}_2$ or $\text{LIG-}\underset{\overset{|}{R}}{\text{NH}}$ for the purposes of this specification.

When the groups LIG and R are together residues of the ligand, they can be separate and distinct residues or bonded together to form a ring.

Preferably the ligand is used in molar excess with respect to its amino groups over the aldehyde groups of the oxidised galactomannan.

The ligand preferably comprises a biological molecule, for example an antigen, antibody, immunoglobulin, metabolite, enzyme serum protein, DNA or an oligonucleotide. Examples of enzymes used are lactate dehydrogenase, invertase and beta-glucosidase. Two or more different ligands can be added to a single galactomannan molecule. Single-stranded DNA could be bonded via the 6-amino group of adenine bases.

The reaction of the aldehyde groups with the amine results in a Schiff's base, probably of partial formula:

$$-\text{C}=\text{N}-\text{LIG} \quad \text{or} \quad -\text{C}=\overset{\overset{R}{|}}{\text{N}^{\oplus}}-\text{LIG}$$

At all events, the reaction product requires reduction by a reducing agent such as sodium cyanoborohydride, to displace the reaction equilibrium in favour of the desired product.

The binding partner used in the affinity chromatography will be chosen appropriately. For example the amine serotonin can be immobilised on the activated galactomannan and used to purify neuraminic acid or a derivative thereof, protein A can be immobilised and used to purify IgG or vice versa. An immobilised antigen will be useful in purifying an antibody thereto or vice versa. A variety of enzymes can be conjugated to the galactomannan.

The bound partner (ligate) can be recovered from the immobilised ligand in various ways including conventional gel filtration.

The galactomannan can be immobilised by connecting it physically, chemically, preferably chemically, or part physically/part chemically to a solid matrix or by forming a matrix of the galactomannan. In one embodiment of the invention, the galactomannan is reacted with D-galactose oxidase to oxidise only a proportion of the available $-CH_2OH$ groups and these are used to couple the galactomannan to any matrix material having a pendant primary or secondary amino group, for example aminopropyl, $-(CH_2)_3NH_2$ groups. A silicate, e.g. control pore glass or kieselguhr containing aminopropyl groups could be used, for example. The coupling results in a Schiff's base, which is preferably reduced e.g. with sodium cyanoborohydride, in order to provide a linkage of higher stability to pH changes. At least some of the remaining $-CH_2OH$ groups on the galactose residues are then reacted with the D-galactose oxidase to produce aldehyde groups for coupling to the ligand as described above.

The above procedure could be reversed, coupling to the ligand being carried out first, followed by further oxidation of the galactomannan and coupling of the product to the matrix or crosslinking.

It is not necessary for the amino groups to be provided on the fundamental matrix material itself. A fundamental matrix containing, say, carboxyl groups, e.g. as in an acrylic polymer,

can be reacted with a reagent providing amine functional groups. A fundamental matrix of an inorganic oxide such as oxides and hydroxides of silicon, aluminium and titanium could also be modified to introduce pendant amine groups, e.g. aminopropyl groups, by reactions known per se. In another procedure the fundamental matrix is provided with amino groups by causing an auxiliary material containing amino groups to be physically adsorbed thereon or adsorbed thereinto, whereafter the galactomannan is chemically linked to the amino groups of the auxiliary material. Preferably the auxiliary material is a protein, especially BSA. Alternatively the galactomannan could be reacted with an amine providing a functional group for attachment to the matrix. In all cases the reacted residue is regarded, for the purposes of definition herein, as part of the matrix residue. Thus, it is convenient to define the polysaccharide as bound to the matrix through groups of formula

$$-\ G'\ -\ CH_2\ -\ \underset{\displaystyle R}{\underset{|}{N}}\ -\ MAT$$

where G' represents a further pendant or terminal D-galactose residue on the polysaccharide, MAT represents a residue of the matrix and R represents a hydrogen atom or MAT and R together represent a residue of the matrix (somewhat analogously to the previous definition of R and LIG, but it would not be necessary for R and MAT to be part of the same individual molecule). Thus, where a matrix has aminopropyl groups, the -NR- group of the above formula is the -NH- residue of an aminopropyl group and the $-(CH_2)_3-$ linkage is part of the matrix residue MAT.

Conveniently, the oxidised galactomannan is in molar excess with respect to its aldehyde groups over the amino groups of the matrix.

In another embodiment cross-links are produced in the galactomannan, conveniently epoxy cross-links. Epichlorohydrin is a convenient reagent for introducing them, e.g. in a manner which

is known from the above-mentioned literature. Another cross-linking reagent is divinyl sulphone.

The following Examples illustrate the invention.

EXAMPLES

Example 1

(1) Purification of galactomannan

The commercially available form of guar (*Cyamopsis tetragonoloba*) gum was purified by ethanolic precipitation. A 1% (w/v) solution of gum was prepared by adding to the guar the least amount of ethanol required to wet it and allowing the guar to stand for 2 hours, then adding it to distilled water with vigorous stirring. The resulting solution was then heated at 90°C for 30 minutes with further stirring. After cooling, insoluble material was removed by centrifugation (7000 g) for 10 minutes. The supernatant solution was then brought to 50% (w/v) ethanol and the white precipitate collected by straining through muslin. The precipitate was then washed with 96% ethanol and diethyl ether and dried at room temperature.

(2) Immobilisation of galactomannan by cross-linking

Purified guar gum from Stage (1) (10 g) was with vigorous stirring added to 20% (v/v) propan-2-ol (200 ml) containing sodium hydroxide (1.4 g). The mixture was perfused with nitrogen for 1 hour, after which epichlorohydrin (1.5 g) was added. The guar gum was then incubated in a shaking water bath at 40°C for 16 hours, followed by 8 hours at 60°C. The resulting cross-linked guar gum (CL-guar) was brought to pH 7 with hydrochloric acid and suspended in a 1 litre measuring cylinder where very large particles and fines were removed by suspension and settling. The CL-guar was then lyophilized and stored dry.

(3) Activation of CL-galactomannan

CL-guar (0.25 g) was oxidised with galactose oxidase (50 U) and catalase (10,000 U) in 0.1 M phosphate buffer pH 7.0 (12.5 ml) for 24 hours at 25°C. After oxidation the CL-guar was washed with 20 mM sodium bicarbonate (20 ml), and brought to 0.1 M in sodium phosphate buffer.

(4) Conjugation of serotonin (5-hydroxytryptamine) to activated CL-galactomannan

To the oxidised CL-guar from Stage (3), in 0.1 M sodium phosphate buffer pH 7.0 (12.5 ml), serotonin (50 mg) and sodium cyanoborohydride (24 mg) were added and the reduction was allowed to proceed for 36 hours. The product was then washed as described in Stage (3).

(5) Use of the ligand-bearing supports in affinity chromatography

The ligand-bearing support from Stage (4) was put into a syringe barrel to give approximately 2 ml bed volume. Chromatography was carried out using pure fetuin in a starting buffer of 60 mM sodium phosphate buffer pH 7.0. The binding capacity of the column was 2 mg fetuin. Elution was continued until the $OD_{280}$ returned to zero, when fetuin was eluted with starting buffer containing 1 M sodium chloride.

Non-specific binding or proteins to the column was checked using bovine serum alumin (10 mg) and was found to amount to 100 micrograms eluting with fetuin. Fractions containing fetuin were located by absorbance at 280 nm and by measurement of sialic acid.

Example 2

Example 1, Stage (1) was repeated using locust bean (*Ceratonia siliqua*) gum as starting material. Stage (2) was repeated except that 350 ml of the propan-2-ol was used instead of 200 ml.

Example 3

Example 2 was repeated using tara (*Caesulpinia spinosa*) gum as starting material.

Example 4

Example 1, Stage (3) was repeated on twice the scale.

Human IgG (50 mg) in 0.1 M phosphate buffer pH 7.0 (25 ml) and sodium cyanoborohydride (47 mg) were added and the reduction carried out for 48 hours. The product was then washed as described in Example 1, Stage (3). A more preferred method of washing is described in Example 8.

Pure Protein A (Sigma 5 mg) was chromatographed on in a syringe barrel column (2 ml bed volume) of the IgG-coupled support prepared above. The starting buffer was 0.1 M sodium phosphate-0.25 M NaCl pH 8.0. Elution continued until the $OD_{280}$ returned to zero, when Protein A was eluted with 0.1 M glycine buffer pH 2.5. The capacity of this column is at least 5 mg of Protein A. Any further adsorbed material can be washed off with 1 M sodium chloride, to regenerate the column.

Non-specific binding of bovine serum albumin was not detected. Protein A was detected in fractions by Laurell rocket immunoelectrophoresis into IgG.

Example 5

(1) Purification of galactomannan

Purified guar gum was obtained from Example 1, Stage (1).

(2) Covalent attachment of galactomannan to Control Pore Glass (CPG) via aminopropyl groups

Aminopropyl-CPG was prepared using 3-aminopropyl-triethoxysilane and 700 Angstroms pore diameter (200-400 mesh) CPG, using the aqueous method of H.H. Weetall, Methods in Enzymology, 44, pages 134-148, cited above.

Purified guar gum from Stage (1) (0.2 g) was oxidised by adding to it galactose oxidase (50 U) and catalase (10,000 U) for 4 hours at 25°C in 0.1 M sodium phosphate buffer pH 7.0 (50 ml). The solution was then heated to 100°C for 5 minutes and then cooled. Aminopropyl-CPG (1 g) was added, with sodium cyanoborohydride (100 mg). The reduction was allowed to continue for 36 hours at 25°C, after which the CPG-galactomannan conjugate was washed with 20 mM sodium acetate pH 4.5 (50 ml), 1 M sodium chloride (50 ml), 20 mM sodium bicarbonate (50 ml) and then brought back to 0.1 M phosphate buffer. The product was designated "Si-galactomannan".

(3) Activation of Si-galactomannan

The previously prepared Si-galactomannan in 0.1 M phosphate buffer, pH 7.0 (50 ml) was treated with galactose oxidase (50 U) and catalase (10,000 U) for 8 hours at 25°C and then washed as described in Example 1, Stage (3).

(4) Conjugation of IgG to activated Si-galactomannan

The activated Si-galactomannan was then reacted with human IgG (30 mg) and sodium cyanoborohydride (38 mg) in 0.1 M phosphate buffer pH 7.0 (20 ml) for 48 hours and washed as described in Example 1, Stage (3). The resultant IgG-bearing support is useful for purifying protein A in the manner of Example 1, Stage (5).

Example 6

Conjugation of beta-glucosidase to Si-galactomannan

Si-galactomannan (1 g) was prepared as described in Example 5, Stages (2) and (3). The activated Si-galactomannan (1 g) was then reacted with beta-glucosidase (EC 3.2.1.21) (30 mg) and sodium cyanoborohydride (38 mg) in 0.1 M sodium phosphate buffer pH 7.0 (20 ml) for 48 hours and then washed as in Example 1, Stage (3). The total washings were pooled, dialysed against 25 mM sodium acetate pH 5.0 and assayed for beta-glucosidase activity.

Activity of the conjugated beta-glucosidase

One unit of enzyme activity = 1 micromole of p-nitrophenol released from p-nitrophenyl-beta-glucoside per minute at 37$^{o}$C.

Beta-glucosidase activity was measured at 37$^{o}$C in 0.25 M sodium acetate pH 5.0 (0.1 ml) plus p-nitrophenyl-beta-glucoside (0.8 ml of 2 mg/ml) with a known amount of the support material in suspension (up to 0.1 ml). Aliquots (0.1 ml) were removed at 30 second intervals, added to 0.25 M glycine pH 10.0 (3.9 ml) and the absorbance read at 400 nm.

(a) Recovery of enzyme activity in the total washings and conjugated to Si-galactomannan equalled 85% of the starting units.

(b) The starting mixture contained 120 U of beta-glucosidase of which 7.7 U were found to be conjugated to Si-galactomannan (1 g) and 94 U found in the washings.

(c) After 39 days at 20$^{o}$C in 25 mM sodium acetate buffer pH 5.0 the beta-glucosidase conjugated to Si-galactomannan retained 72% of its activity.

Example 7

Conjugation of beta-glucosidase to CL-galactomannan

To the oxidised CL-galactomannan from Example 1, Stage (3) in 0.1 M sodium phosphate buffer pH 7.0 (12.5 ml), beta-glucosidase (EC 3.2.1.21) (25 mg) and sodium cyanoborohydride (24 mg) were added and the reduction allowed to proceed for 48 hours. The product was then washed as in Example 1, Stage 3, the washings were then pooled, dialysed against 25 mM sodium acetate buffer pH 5.0 and assayed for beta-glucosidase activity.

Activity of the conjugated beta-glucosidase

(a) Control studies of beta-glucosidase under the conditions used for coupling showed no loss of beta-glucosidase activity (measured as in Example 6) in the presence or absence of sodium cyanoborohydride over 48 hours as compared with the original enzyme activity.

(b) The recovery of enzyme activity in the washings and conjugated to the support equalled 80% of the starting units.

(c) The starting mixture contained 175 U of beta-glucosidase of which 134 U were found in the washings and 6 U coupled to CL-galactomannan (250 mg).

(d) After 24 days at 20$^{o}$C in 25 mM sodium acetate, pH 5.0 the beta-glucosidase conjugated to CL-galactomannan retained 75% of its activity.

Example 8

(1) Purification of galactomannan

Purified guar gum was obtained from Example 1, Stage (1).

(2) Covalent attachment of galactomannan to control pore glass

This procedure makes use of the stable adsorption of protein (bovine serum albumin) onto control pore glass (CPG), followed by use of free amino groups of the protein to bond oxidised galactomannan to the protein-CPG matrix.

Control pore glass was washed in a column with excess BSA (10 mg/ml) until BSA was present in the eluate, i.e. the column saturated. The CPG was then washed with 1 M NaCl until no protein could be detected in the eluate. This CPG-BSA complex was

then treated as in Example 5, Stage (2), to produce a galactomannan-BSA-CPG complex the galactomannan being covalently linked to the BSA, yielding approximately 2 mg galactomannan/g CPG.

(3) Activation of the galactomannan-BSA-CPG complex

The complex from Stage (2) was treated with galactose oxidase and catalase as described in Example 5, Stage (3).

(4) Conjugation of invertase to the support material

An excess of invertase (1000 U) was added to the support material from Stage (3) in 0.1 M acetate buffer pH 5.0 (5 ml) containing $NaCNBH_3$ (9.5 mg). After 48 hours at room temperature ($20^oC$) the support was washed three times with 0.1 M sodium acetate buffer, pH 4.0, containing 0.5 M sodium chloride, then unbuffered 1 M sodium chloride, followed by 0.1 M phosphate buffer pH 8.0 containing 0.5 M sodium chloride. This method of washing removes any "loose" galactomannan, i.e. any which is not covalently bonded to its fundamental matrix. Of course, since a ligand such as invertase is conjugated to the galactommannan, ligand conjugated to the "loose" galactomannan will also be removed. The method of washing is also preferred to use with IgG, to that described in Examples 4 and 5.

Invertase was coupled to the above complex support material and the resultant conjugate was found to contain 11 U of invertase activity/g of support material.

Example 9

(1) Purification of galactomannan

Purified guar gum was obtained from Example 1, Stage (1).

(2) Covalent attachment of galactomannan to another polysaccharide

Chitin (poly-N-acetyl-glucosamine) can be partially de-acylated with aqueous sodium hydroxide to produce free amino groups on the polysaccharide backbone which can be used to bond oxidised galactomannan. Chitin (2 g) heated for 2 hours at $100^oC$ in 4 M sodium hydroxide (20 ml) had sufficient $NH_2$-groups to couple 5 mg of galactamannan per gram of chitin using the procedure of Example 5, Stage (2).

(3) Activation of the galactomannan-chitin reaction product

The product from Stage (2) was treated with galactose oxidase and catalase as described in Example 5, Stage (3).

(4) Conjugation of invertase to the support material

Invertase was coupled to the support material from Stage (3) as described in Example 8 and the resultant conjugate was found to contain 26 U of invertase activity/g of support.

CLAIMS

1. A support material comprising an immobilised polysaccharide having pendant or terminal groups of general formula

$$-\ G\ -\ CH_2\ -\ \underset{\substack{|\\ R}}{N}\ -\ LIG$$

wherein:

G represents a D-galactose residue
LIG represents a ligand residue, and
R represents a hydrogen atom,
or LIG and R together represent a residue of the same ligand molecule.

2. A support material according to Claim 1 wherein the polysaccharide is a cross-linked polysaccharide.

3. A support material according to Claim 1 wherein the polysaccharide is bound to a glass matrix.

4. A support material according to Claim 3 wherein the polysaccharide is bound to the matrix through groups of formula

$$-\ G'\ -\ CH_2\ -\ \underset{\substack{|\\ R}}{N}\ -\ MAT$$

where G' represents a further pendant or terminal D-galactose residue on the polysaccharide, MAT represents a residue of the matrix and R represents a hydrogen atom or MAT and R together represent a residue of the matrix.

5. A support material according to Claim 4 wherein the matrix has aminopropyl groups thereon, the -NR- group shown in Claim 4 being the -NH-residue of an aminopropyl group.

6. A support material according to any preceding claim wherein the polysaccharide is a <u>D</u>-galacto-<u>D</u>-mannan.

7. A process for preparing a support material claimed in Claim 1, which comprises

(a) immobilising a polysaccharide having pendant or terminal non-reducing D-galactose residues thereon

(b) activating the immobilised polysaccharide by oxidation of $-CH_2OH$ groups present in the D-galactose residues by D-galactose oxidase, and

(c) reacting the activated immobilised polysaccharide with a ligand containing a primary or secondary amino group to form a Schiff's base (aldo-imine or -imminium ion), and with a reducing agent to reduce the Schiff's base.

8. A process according to Claim 7 wherein the immobilisation is carried out by cross-linking the polysaccharide.

9. A process according to Claim 7 wherein the immobilisation is carried out by partial oxidation of the non-reducing D-galactose residues of the polysaccharide by D-galactose oxidase, with removal of the hydrogen peroxide formed, and reacting the oxidised polysaccharide with a matrix having a primary or secondary amino group or with a linking reagent having such an amino group and another group reactable with a matrix, followed by reacting the reactable group with the matrix, said reaction being carried out to form a Schiff's base, and further, if desired, reducing the Schiff's base.

10. A process of affinity chromatography which comprises chromatographing on a support material claimed in any one of Claims 1 to 6 or prepared by a process claimed in any one of Claims 7 to 9, a binding partner for the ligand.

European Patent Office

**0165800**

Application number

# EUROPEAN SEARCH REPORT

| DOCUMENTS CONSIDERED TO BE RELEVANT | | | EP 85304328.9 |
|---|---|---|---|
| Category | Citation of document with indication, where appropriate, of relevant passages | Relevant to claim | CLASSIFICATION OF THE APPLICATION (Int Cl 4) |
| D,X | Carbohydrate Research, vol. 81, no. 1, May 1, 1980, Amsterdam<br>L.D.HALL et al. "A high-yielding, specific method for the chemical derivatization of D-galactose-containing polysaccharides: oxidation with D-galactose oxidese, followed by reductive amination" pages C10-C12<br>* Totality *<br>-- | 1,6 | C 08 B 37/00<br>B 01 J 20/24<br>G 01 N 30/48<br>C 12 N 11/10<br>C 12 Q 1/54<br>B 01 D 15/08 |
| A | <u>DE - A1 - 2 840 503</u> (INSTITUT MERIEUX)<br>* Claims *<br>-- | 1,2,3,10 | |
| D,A | FEBS Letters, vol. 68, no. 1, September 1976, Amsterdam<br>J.LÖNNGREN et al. "Cross-linked Guaran: Aversatile Immunosorbent for D-Galactopyranosyl binding lectins" pages 31-34<br>* Introduction *<br>---- | 1,10 | TECHNICAL FIELDS SEARCHED (Int Cl 4)<br>B 01 D<br>B 01 J<br>C 08 B<br>C 12 N<br>C 12 Q<br>G 01 N |

The present search report has been drawn up for all claims

| Place of search | Date of completion of the search | Examiner |
|---|---|---|
| VIENNA | 05-09-1985 | BECKER |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document